# EUROPEAN PATENT APPLICATION

(11) **EP 3 086 249 A1**
(43) Date of publication of application: **26.10.2016**
(21) Application number: 16165450.4
(22) Date of filing: 14.04.2016
(51) Int. Cl.: G06F 19/00

(54) **MEDICATION MANAGEMENT SYSTEM**

(30) Priority: 24.04.2015 US 201514695364
(71) Applicant: Hand Held Products, Inc., Fort Mill, SC 29707 (US)
(72) Inventor: WILZ, Sr., David, Morris Plains, NJ New Jersey 07950 (US); WALCZYK, Joseph A., Morris Plains, NJ New Jersey 07950 (US); MEAGHER, Mark, Morris Plains, NJ New Jersey 07950 (US)
(74) Representative: Houghton, Mark Phillip

(57) **Abstract**

A method of medication management involves capturing an image depicting a machine readable code symbol, where the code symbol associates a medication with a patient. At a programmed processor: determining prescribing information for the medication and the patient; determining that a dose of the medication is due; and generating an alert indicating that the dose of medication dosage is due to be taken by the patient.

## Description

### FIELD OF THE INVENTION

The present invention relates to utilizing machine readable indicium to assist in reminding a patient and monitoring and dispensing of medication in a home environment.

### BACKGROUND

For home medication use, the taking of medication is often done by the consumer without any real knowledge of potential side effects, restrictions, combinations of medicines or medicines with food that have dangerous interactions, etc. Additionally, some people, in particular the elderly, often have difficulty remembering to take their medications or difficulty remembering if they have already taken a medication.

Therefore, a need exists for a mechanism to facilitate the proper home administration of medications.

### SUMMARY

Accordingly, in one aspect, the present invention embraces a system and method for reading code symbols such as bar codes that relate a patient with medication information and provides patient alerts. In some implementations, the system dispenses or otherwise provides a patient with medication access when medication is due and creates a log of relevant events that permit monitoring of the patient's compliance with a treatment regimen.

In an exemplary embodiment, a system for medication management includes a camera and a user interface having a visual display. One or more processors are communicatively coupled to the camera and the user interface system with the one or more processors being configured to: capture from the camera an image depicting a machine readable code symbol, where the code symbol associates a medication with a patient; determine prescribing information for the medication and the patient; determine that a dose of medication is due; and generate an alert indicating that the dose of medication is due to be taken by the patient.

In certain implementations, a medication dispenser is provided, and the one or more processors are further configured to cause the medication dispenser to provide patient access to a dose of medication when the medication is due to be taken by the patient. In certain implementations, the one or more processors are further configured to create entries to a log that record times and instances of alerts and dispenses of medication.

In certain implementations, a medication dispenser is provided, and the one or more processors are further configured to: authenticate the presence of the patient; and cause the medication dispenser to provide patient access to a dose of medication when the medication is due to be taken by the patient and when the patient is authenticated.

In certain implementations, the one or more processors are further configured to create a log entry that records times and instances of alerts, authentications, and medication dispenses. In certain implementations, the one or more processors are further configured to: retrieve information relating to the medication; and cause the user interface display to display the information relating to the medication. In certain implementations, the information is retrieved from a database residing in the one or more processors' directly accessible memory. In certain implementations, the information is retrieved from a database residing outside the one or more processors' directly accessible memory In certain implementations, the database is accessed via a wireless communication system. In certain implementations, the one or more processors are further configured to: receive acknowledgements of alerts by the patient; create a log entry that records time and instances of alerts and acknowledgements of alerts. In certain implementations, the code symbol comprises a bar code.

In another example embodiment, a system for medication management, includes a camera and a medication dispenser. A user interface with a visual display is provided. One or more processors are communicatively coupled to the camera and the user interface system, the one or more processors being configured to: capture from the camera an image depicting a machine readable bar code symbol, where the bar code symbol associates a medication with a patient; determine prescribing information for the medication and the patient; determine that a dose of medication is due; generate an alert indicating that the dose of medication is due to be taken by the patient; retrieve information from a database relating to the medication; cause the user interface display to display the information relating to the medication; authenticate the presence of the patient; cause the medication dispenser to provide patient access to the dose of medication when the medication is due to be taken by the patient and when the patient is authenticated; and create a log entry that records times and instances of alerts, authentications, and medication dispenses.

In another example embodiment, a method of medication management involves: at a camera, capturing an image depicting a machine readable code symbol, where the code symbol associates a medication with a patient; at one or more programmed processors: determining prescribing information for the medication and the patient; determining that a dose of the medication is due; and generating an alert indicating that the dose of medication is due to be taken by the patient.

In certain implementations, the one or more processors cause a medication dispenser to provide patient access to a dose of medication when the medication is due to be taken by the patient. In certain implementations, the one or more processors creating entries to a log that records times and instances of alerts and dispenses of medication. In certain implementations, the one or more processors authenticate the presence of the patient and cause a medication dispenser to provide patient access to a dose of medication when the medication is due to be taken by the patient and when the patient is authenticated In certain implementations, the one or more processors create a log entry that records times and instances of alerts, authentications, and medication dispenses.

In certain implementations, the one or more processors retrieve information relating to the medication and cause the user interface display to display the information relating to the medication. In certain implementations, the code symbol comprises a bar code.

Another example embodiment of a system for medication management has a camera and a user interface with a visual display. One or more processors are communicatively coupled to the camera and the user interface system with the one or more processors being configured to: capture from the camera an image depicting a machine readable bar code symbol, where the bar code symbol associates a medication with a patient; and decode the bar code symbol to determine prescribing information for the medication and the patient.

Another example method of medication management involves: at a camera, capturing an image of text residing on a medication container which associates a medication and prescribing information with a patient; at one or more programmed processors: processing the image of text to interpret the medication and prescribing information for the patient; determining that a dose of the medication is due; and generating an alert indicating that the dose of medication is due to be taken by the patient.

The foregoing illustrative summary, as well as other exemplary objectives and/or advantages of the invention, and the manner in which the same are accomplished, are further explained within the following detailed description and its accompanying drawings.

### BRIEF DESCRIPTION OF THE DRAWINGS

Figure 1 depicts an example of a patient identification card consistent with certain embodiments of the present invention.
Figure 2 depicts another example of a patient identification card consistent with certain embodiments of the present invention.
Figure 3 depicts an example medication bottle carrying a bar code consistent with certain embodiments of the present inventions.
Figure 4 is an example block diagram depicting a medication management system/device consistent with certain embodiments of the present invention.
Figure 5 is an example block diagram depicting a variation of the medication management system consistent with certain embodiments of the present invention.
Figure 6 is an example block diagram depicting a medication management system including biometric patient identification and medication dispenser consistent with certain embodiments of the present invention.
Figure 7 is a flow chart depicting an example process consistent with certain embodiments of the present invention.
Figure 8 is another flow chart depicting an example process consistent with certain embodiments of the present invention.
Figure 9 depicts an example block diagram showing alternatives that can be used for authentication consistent with certain embodiments of the present invention.
Figure 10 schematically illustrates an example medication dispenser consistent with certain embodiments.

### DETAILED DESCRIPTION

The present invention embraces a device and method that reads bar code data or similar indicium associated with a medication in order to assist a patient or caregiver in assuring proper dosing of medication in a home environment and further allows the patient easy access to information about the medication(s). In certain implementations, such a method includes, at a camera, capturing an image depicting a machine readable code symbol, where the code symbol associates a medication with a patient. At a programmed processor: determining prescribing information for the medication and the patient; determining that a dose of the medication is due; and generating an alert indicating that the dose of medication dosage is due to be taken by the patient.

A bar code is a machine readable optical indicium that contains information relating to an object - generally an object on which the code is affixed. Bar codes are a popular class of machine readable optical indicia that are generically referred to herein as code symbols. The original bar codes, which are still very widely used, encode data using a series of lines having varying widths and spacing to encode the data. Such bar codes may be referred to as a one dimensional bar code. Other bar codes utilize rectangles, dots or other shapes for optically encoding the data. A matrix bar code (or two dimensional bar codes such as the QR code) is a type of bar code that can hold more information than conventional bar codes. For purposes of this document, each of the above types of bar codes is generically referred to as a bar code.

As previously noted, for home medication use, the taking of medication is often done by the consumer without any real knowledge of potential side effects, combinations of medicines or medicines with food that have dangerous interactions, restrictions/precautions (e.g., not to operate machinery or make important decisions) etc. Additionally, people often have difficulty remembering to take their medications or difficulty remembering if they have already taken the medication. By using optical scanning technology, errors in administration of medication can be minimized.

In accord with certain embodiments, scanning technology can be used to either scan an encoded symbol on a medicine container (e.g., a prescription medicine bottle) or scanning a patient identification (ID) card or both in order to assist in regulating the administration of the medication, to actually dispense the medication, to retrieve information about the medication and its use, and/or to provide documentation of the patient's compliance with the prescribing doctor's treatment. Greater safety be achieved when the patient is educated about the medications being taken and is assisted in complying with the treatment prescription.

In accord with the present teachings, those skilled in the art will appreciate that there are many variations in which a system can be configured and operated. The scanning system involved could operate in many configurations including, for example, the following two examples.

In a first example, a system tracks and controls the medicine by use of a patient ID card when the card is scanned or when a patient is otherwise identified, for example by use of biometric identification. Upon identification of the correct patient, the medicine prescribed can be automatically dispensed. This example utilizes an initial proper setup of the system to input the medications and prescribing details for all medicines taken by the patient. The system can then assist in preventing duplicate taking of medicine by withholding double doses or alerting the patient that a dose is not due or has already been taken. The system can also have an alarm mechanism to alert the consumer to take the medication. Additionally the system can generate and save a historical log of all medications taken by said consumer and other details of significant actions. Such log can be available to health care workers and caregivers to monitor compliance with the treatment.

In a second example, the system can be coupled to the Internet to get significant information on the medications being taken. Scanning a patient ID card or a coded medicine bottle can produce an appropriate code or link (e.g., a Universal Resource Locator - URL) that accesses side effects, food and drug interactions, restrictions/precautions, alternative medications, etc. as they relate to the patient and/or a particular medication.

In another variation, the system can be programmed by scanning a code on the medication bottle. This code could contain warnings, the dosage and frequency of use, the number of refills, etc. When connected to the Internet, refills could be ordered or an appointment with the patient's physician could be scheduled or the user could be alerted to make such appointment. The device/system alerts can be communicated to the user via a text message (i.e., a Short Message Service - SMS messaging), email, or through audible and visual alerts from the device.

In accord with the first example above, an initial setup is conducted to enter all medications and prescribing instruction for the patient. Such initial setup can be assisted by utilizing the patient ID card and/or the patient's bar coded medication containers, or can be entered via a manual entry system, or download from an external database or from another dispensing device, etc. The patient then scans their ID card when they want to take the medication.

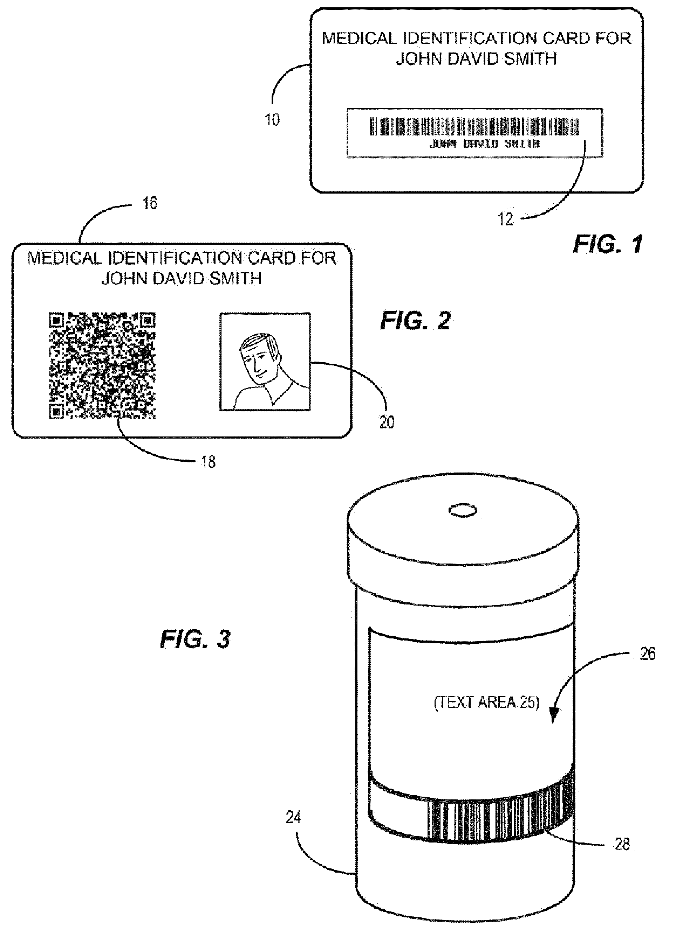

A first example of a patient ID card 10 is depicted in FIG. 1. This example ID card includes the patient name and a conventional one dimensional bar code 12. The patient ID card can also include other items such as a photograph of the patient, insurance information, etc. This information can be provided in printed form and/or encoded via one or more bar codes, for example.

FIG. 2 depicts a second example of an ID card 16 having a QR code 18 (a two-dimensional bar code) and a photograph 20 of the patient. It is noted that that other types of machine readable indicia can be used besides bar codes. Two-dimensional bar codes can encode much larger quantities of data and may directly provide patient information. One-dimensional bar codes or two-dimensional bar codes can also include links to information that can be retrieved over the Internet. Other variations, such as using near field communication and smartcards, will occur to those skilled in the art upon consideration of the present teachings.

In the second example mentioned above, scanning a bar code on a medicine bottle or other medication package provides for information transfer relating to the particular medication. The information can be displayed directly on a display on the scanning system or downloaded automatically to an email or SMS message that can be registered in the system. The information stored in the code, tied to the medication in the device, or downloaded from the internet (dosage, side effects, instructions, etc.) can be attached/embedded/added to a text message or email that can be sent to a stored email address or phone number. FIG. 3 depicts an example medication bottle 24 having a label 26 that is provided with a text area 25 and a bar code 28 that either directly identifies the medication or provides a link to information about the medication. The bar code may directly include prescribing directions or may link to such directions.

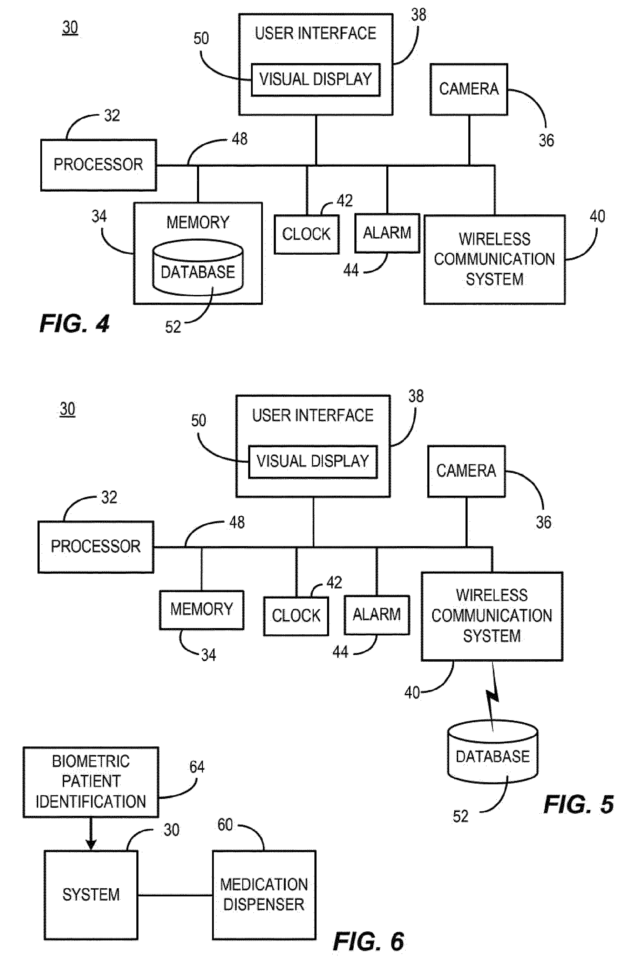

Referring now to FIG. 4, this drawing depicts an example embodiment of a device or system 30 consistent with the present teachings. FIG. 1 schematically depicts an example device 30 in accordance with the present disclosure. The device 30 includes a processor 32, a memory 34, a camera 36, a user interface 38, a wireless communication system 40, a clock 42 and an alarm 44. While processor 32 is shown as a single processor, those skilled in the art will appreciate that multiple cooperative processors (i.e., two or more processors and multiple processor cores) can be utilized to implement certain implementations. Hence, the depiction of a single processor 32 is for convenience of illustration and represents one or more processors.

The processor 32 is communicatively coupled to the memory 34, the camera 36, the user interface 38, the wireless communication system 40, clock 42 and alarm 44 via one or more system buses 48. Alarm 44 may simply represent an alarm process consistent with the teachings herein or may incorporate a speaker, vibrator or other mechanism utilized to get the attention of the patient.

Exemplary devices may include a system bus 48 and/or one or more interface circuits (not shown) for coupling the processor 32 and other components to the system bus 48, and for coupling external components to the system (e.g., a computer, printer, dispenser interface, biometric scanner interface, etc.). In this regard, the processor 32 may be communicatively coupled to each of the other components via the system bus 48 and/or the interface circuits. Similarly, the other components (e.g., the memory 34, the camera 36, the user interface 38, the wireless communication system 40, the clock 42 and the alarm 44) may each be communicatively coupled to other components via the system bus 48 and/or the interface circuits as described above. Other embodiments of system bus architecture providing for efficient data transfer and/or communication between the components of the device may also be employed in other embodiments in accordance with the present disclosure.

The processor 32 is configured to execute programming instructions and to carry out operations associated with the device 30. For example, using instructions retrieved from the memory 34 (e.g., a memory block and/or storage device), the processor 32 may control the reception and manipulation of input and output data between components of the device 30. The processor 32 may operate using an operating system to execute computer code and produce and use data. The operating system, other computer code, and data may reside within the memory 34 that is operatively coupled to the processor 32. The memory 34 generally provides a place to store computer code and data that are used by the device 30. The memory 34 may include Read-Only Memory (ROM), Random-Access Memory (RAM), flash memory, a hard disk drive, solid state drive and/or other non-transitory storage media. The operating system, other computer code (such as code that can be generated using the flow charts and discussion herein for guidance), and data may also reside on a removable non-transitory storage medium that is loaded or installed onto the device 30 when needed. Exemplary removable non-transitory storage media include CD ROM, PC-CARD, flash memory, memory card, floppy disk, magnetic stripe and/or magnetic tape.

The user interface system 38 includes one or more components capable of interacting with a user (e.g., receiving information from a user or outputting information to a user). As depicted in FIG. 4, the user interface system 38 includes a visual display 50. In certain implementations, the visual display 46 can be implemented as a touchscreen display, which is capable of displaying visual information and receiving tactile commands from a user (e.g., selections made by touching the screen with a finger or a stylus, by manipulating a virtual keyboard or virtual control buttons, by painting at a desired selection, or by looking at a desired selection for a predefined period of time). In addition to the visual display 50, the user interface system 38 may also include one or more speakers, buttons, keyboards, mice or other pointing devices, and/or microphones. Entries and interactions with the system 30 can be carried out using touch commands, keyboard, etc. as mentioned above and may also include voice commands that can be interpreted using voice recognition functions forming a part of the user interface and implemented using processor 32. Other variations of interaction with the user interface 38 will occur to those skilled in the art upon consideration of the present teachings.

As noted, the device 30 may include a wireless (or wired) communication system 40. The wireless communication system 40 enables the device 30 to communicate with a wireless network, such as a cellular network (e.g., a GSM network, a CDMA network, or an LTE network), a local area network (LAN), and/or an ad hoc network or a Bluetooth™ network, for example.

The camera 36 may be any device that is able to capture still photographs and/or video. The camera 36 may be capable of capturing both still photographs and video. Although FIG. 4 depicts the device 30 as having a single camera 36, it is within the scope of the present teachings for the mobile device 30 to include more than one camera without limitation. It is further possible to implement the present device utilizing a cellular telephone or tablet computer or other mobile computing device without limitation.

Thus, in certain embodiments, a system for medication management has a camera 36 and a user interface 38 having a visual display. A processor 32 is communicatively coupled to the camera 36 and the user interface 38, with the processor 32 being configured to capture from the camera 36 an image depicting a machine readable bar code symbol, where the bar code symbol associates a medication with a patient, and decode the bar code symbol to determine prescribing information for the medication and the patient.

The processor 32 is in communication with a database 52. As depicted in FIG. 4, the database 52 may be stored locally within the memory 34 for direct access in this embodiment so that the processor 32 can access the local database 52.

In one embodiment consistent with the present teachings, a user can scan a medicine bottle carrying a bar code or similar indicium and the system 30 can provide a simple yes or no type response that means that it is or is not time to take the next dose of medication. More involved mechanisms of operation are discussed later.

Referring to FIG. 5, another example implementation is depicted. In this alternative embodiment, the processor 32 may access the database 52 via the wireless communication system 40. In other words, the processor may access the database 52 residing outside the device 30 either through the internet or a local area network via the wireless communication system 40.

In either example, the database 52 may include information associated with a code symbol whether patient information, medication or a combination thereof. In either of these embodiments, the user can utilize the camera 36 to scan or read a bar code or other machine readable indicia so as to either derive patient and/or medication related information, or retrieve related information from the database 52 or to a specified Internet location that provides such information or some combination thereof. In the case of a patient ID card, a patient ID card bar code may be imaged by the camera 36 and processor 32 can then determine the patient from unique identifiers encoded in the bar code. This information can then be linked to information either in the database 52 or to information on the Internet to carry out management of the patient's medication and related information.

In the case of a medicine container, the bar code thereon can be similarly imaged by the camera 36 and the prescribing information entered into the database 52. Once the prescribing information is entered, the processor can utilize the clock 42 to determine when the next dosage is due and can provide any suitable alarm 44 (audible, visual, tactile, email, text message, etc. or combination thereof) to remind the patient that it is time to take the medication. A further scan of the medicine container can be used as a way for the patient to determine if it is near time for taking the medication, or to otherwise find out information about the medication including medicine or food interactions, restrictions/precautions, side effects, etc. Additionally, when the medication is nearly used up, the number of doses can be tracked by the processor 32 and a request for a refill can be dispatched to the pharmacy or doctor's office for processing. Such requests can be effected via an email, fax or other electronic communication and/or by use of an alert to the patient or a caregiver so that the patient or caregiver can make arrangements for the refill.

Thus, an example system 30 for medication management consistent with the present teachings, has a camera 36 and a user interface 38, where the user interface includes a visual display 50. Processor 32 is communicatively coupled to the camera 36 and the user interface 38, with the processor 32 being programmed to capture an image using the camera 36 with such image depicting a machine readable code symbol such as a bar code, where the code symbol associates a medication with a patient. The processor 32 further determines prescribing information for the medication and the patient, and determines that a dose of medication is due. The processor 32 generates an alert indicating that the dose of medication dosage is due to be taken by the patient.

In a further embodiment consistent with the present teachings, the system/device 30 can include or be connected to a medication dispensing device 60. In such case, the patent's medication is dispensed at appropriate times via the dispenser 60. In one example, when the time for dispensing a medication arrives, the patient is alerted in a suitable manner. The patient can then authenticate to the system, for example by a scan of the patient ID card and/or biometric identification device 64 or by entry of a password or PIN number, etc. Once the patient is suitably authenticated, the medication can be dispensed to the patient. In this manner, medication is only dispensed in controlled amounts when the user is properly identified and when it is time for the medication to be taken.

While 60 and 64 are shown attached to system 30, these elements can be integral with system 30 or may be add-on modules or separately interconnected components without limitation. Thus, in accord with further embodiments, the system 30 may include a medication dispenser 60, with the processor 32 being further programmed to cause the medication dispenser 60 to provide patient with access to a dose of medication when the medication is due to be taken by the patient. This can be done by dispensing the medication through a chute or the like or by simply unlatching a compartment giving the patient access to a dose of the medication.

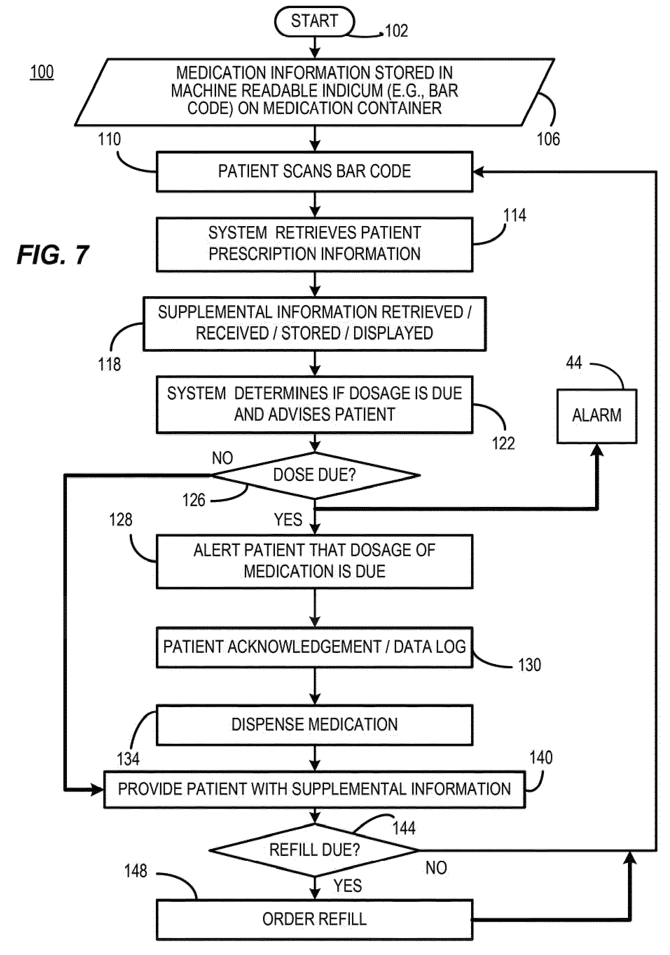

One example process 100 consistent with the present teachings is shown in FIG. 7 starting at 102. In this example, medication and/or patient information is first stored in the system 30 either by download, manual entry, reading prescription bottles or by reading a patient ID card or some combination thereof at 106. After this point, it is assumed for this discussion that the patient and medication information is in the system for use by the patient.

At 110, the patient scans a machine readable indicium such as a bar code. The system 30 reads the indicium and determines information about the patient and the medication from the database 52 and may supplement that information using for example wireless 4G communication over the Internet. The patient prescription information is retrieved at 114 and may present information to the patient regarding a particular medication or about all of the patient's prescriptions.

For example, in one embodiment, the user scans a medicine bottle bar code at 110. This causes the processor 32 to retrieve relevant prescribing information about the particular medication's use by this patient for the medication in the scanned bottle. At 118, processor 32 can retrieve other supplemental information about the patient and the medication for use by the patient. At 122 the processor 32 can initiate an inquiry of a repository for the patient's prescribing information to determine if it is an appropriate time (e.g., within a suitable time window) for the patient to take the next dose of the medication. If so at 126, the patient may be alerted that it is time for the next dosage of medication at 128. Such alert can desirably be generated with or without the patient initiating an action by scanning the medicine bottle. In the case of no patient initiation, an alarm is generated by alarm 44 which operates by use of the processor 32 in cooperation with clock 42. This alarm causes generation of a patient alert at 128 to notify the patient that it is time to take the medication. Alarm 44 may also be used to advise the patient of important information regarding the medication (e.g., take with food, lie down for 30 minutes after taking, new FDA warning, interaction warning, etc.).

In response to the patient alert, the patient may scan the medicine bottle or patient ID card to acknowledge the alert and stop any audible, visual or tactile alerts at 130. In other embodiments, the patient may acknowledge an alert by reply email or text, entry of a password or other code, or entry of an acknowledgement into the user interface. The processor 32 may log this event for later retrieval by the patient or medical personnel or other caregivers to assure that the patient is being compliant with the prescription. So, for example, the processor 32 may be further programmed to create entries to a log file in memory 34 that records times and instances of alerts and acknowledgements of the alerts.

In the example of FIG. 6, the acknowledgement by the patient can be by the ID card or by a biometric input or both. In response thereto, a medication dispenser 60 can dispense or otherwise allow access to the medication that is due at the time at 134. This can be accomplished by pre-loading compartments in the dispenser with the medication and then either unlocking the compartments for the patient to access or by releasing the appropriate medication through a chute or other dispensing mechanism without limitation.

In this instance, the processor 32 can be further programmed to cause the medication dispenser 60 to provide patient access to a dose of medication when the medication is due to be taken by the patient at 130. In certain examples, the processor 32 can be programmed to create a log entry that records times and instances of alerts, authentications, and medication dispensing actions or any other significant actions such as scans, queries, data input, data changes, power loss, etc. in memory 34 or to a remote storage location via the wireless communication system 40.

Processor 32 can cause the user interface to display the medicine name, prescribing information (e.g., take one tablet three times per day), and other relevant information that that was retrieved and which the patient may be interested in such as side effects, medication interactions, the intended function of the medication (e.g., lowering blood pressure) and the like. Such supplemental information may be retrieved every time the patient scans a medication bottle or ID card, or can be retrieved once and stored locally for later display. At 140, the patient can be given the option of displaying supplemental information or not. Display 50 can also display a somewhat static image depicting medication dosages and times along with an indication that a particular dose has or has not been taken (i.e., by virtue of an acknowledgement to an alert by the patient, etc.).

Whenever a medication is dispensed (or at any other suitable time) at 60 the processor can determine if it is an appropriate time for the medication to be refilled at 144. If so, the process implements a mechanism to obtain a refill. This can be as simple as displaying an alert for the patient to remind the patient to order a refill. Such reminder can include a number of doses remaining until the medication is gone and indicate if refills require a doctor visit or contact. In other embodiments, the system 30 can initiate a request of the pharmacy or doctor for the refill using any suitable communication mechanism (email, fax, text message, etc.). If it is not time for a refill, the process returns to 110 to await the next scan while the alarm is armed to provide alerts when the next dose is due.

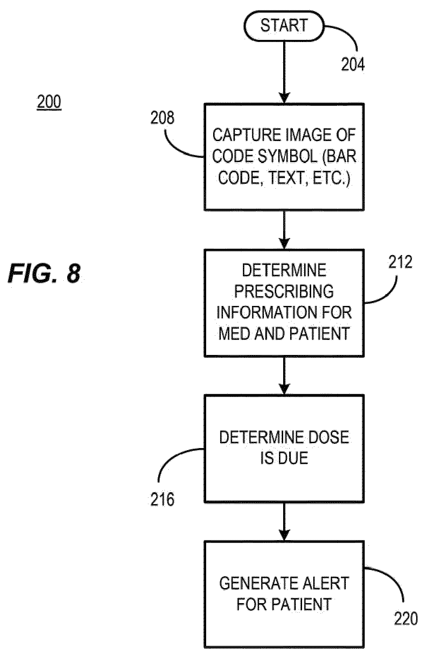

Figure 8 depicts another example method of medication management consistent with the present teachings starting at 204. At 208 a camera captures an image depicting a machine readable code symbol, where the code symbol associates a medication with a patient. A programmed processor then: determines prescribing information for the medication and the patient at 212 and determines at 216 that a dose of the medication is due. At 220, the processor generates an alert indicating that the dose of medication dosage is due to be taken by the patient.

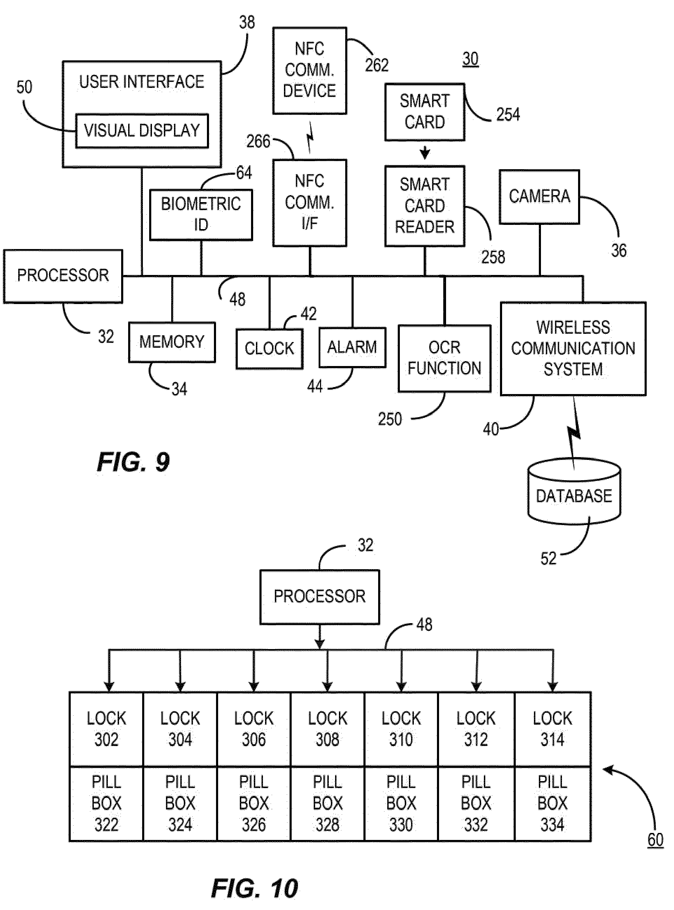

Referring now to Fig. 9, another example implementation is depicted in which camera 36 is used to read indicia in the form of machine readable text that is processed by an OCR function 250 that may be implemented by processor 32 or by a separate OCR processor. In this example, the medication container need not be encoded with bar codes. The prescribing text appearing on the medicine container can be scanned and parsed to determine what the medication is and how it is to be taken. Additionally, this example can utilize other forms of identification to achieve authentication. Examples include devices such as smart cards 254 that can be read by smart card reader 258; or near field communication (NFC) enabled devices such as NFC enabled smart phones 262 can be read by an NFC communication interface 266; or using facial recognition through camera 36 and/or voice recognition through a microphone at the user interface 38. Those skilled in the art will appreciate that any or all of the authentication mechanisms described may be included singly or in combination without departing from examples consistent with the present teachings.

With reference to Fig. 9, an example of pill box dispenser 60 is schematically represented with processor 32 controlling a plurality of electrically controlled locks 302, 304, 306, 308, 310, 312 and 314. Such locks are operative to respectively control compartments of pill storage box 322, 324, 326, 328, 330, 332 and 334 respectively under control of processor 32 via bus 48 or any other suitable communication bus. As shown, seven days (one week) of medication compartments are illustrated. Processor 32 unlocks each of the pill box compartments sequentially at an appropriate day and time to dispense the medication for a particular day. In other embodiments, multiple compartments are provided so that medications can be dispensed throughout the day at the appropriate times. When it is time for the medication to be dispensed, processor 32 controls one of the locks 302-314 to unlock the lock and either open or allow access to the contents of the associated pill box compartment 322-334. In other implementations, when the compartment is unlocked, the contents are dropped through a chute to a reservoir where the patient can retrieve the medication.

Many variations will occur to those skilled in the art upon consideration of the present teachings.

To supplement the present disclosure, this application incorporates entirely by reference the following commonly assigned patents, patent application publications, and patent applications:
U.S. Patent No. 6,832,725; U.S. Patent No. 7,128,266;
U.S. Patent No. 7,159,783; U.S. Patent No. 7,413,127;
U.S. Patent No. 7,726,575; U.S. Patent No. 8,294,969;
U.S. Patent No. 8,317,105; U.S. Patent No. 8,322,622;
U.S. Patent No. 8,366,005; U.S. Patent No. 8,371,507;
U.S. Patent No. 8,376,233; U.S. Patent No. 8,381,979;
U.S. Patent No. 8,390,909; U.S. Patent No. 8,408,464;
U.S. Patent No. 8,408,468; U.S. Patent No. 8,408,469;
U.S. Patent No. 8,424,768; U.S. Patent No. 8,448,863;
U.S. Patent No. 8,457,013; U.S. Patent No. 8,459,557;
U.S. Patent No. 8,469,272; U.S. Patent No. 8,474,712;
U.S. Patent No. 8,479,992; U.S. Patent No. 8,490,877;
U.S. Patent No. 8,517,271; U.S. Patent No. 8,523,076;
U.S. Patent No. 8,528,818; U.S. Patent No. 8,544,737;
U.S. Patent No. 8,548,242; U.S. Patent No. 8,548,420;
U.S. Patent No. 8,550,335; U.S. Patent No. 8,550,354;
U.S. Patent No. 8,550,357; U.S. Patent No. 8,556,174;
U.S. Patent No. 8,556,176; U.S. Patent No. 8,556,177;
U.S. Patent No. 8,559,767; U.S. Patent No. 8,599,957;
U.S. Patent No. 8,561,895; U.S. Patent No. 8,561,903;
U.S. Patent No. 8,561,905; U.S. Patent No. 8,565,107;
U.S. Patent No. 8,571,307; U.S. Patent No. 8,579,200;
U.S. Patent No. 8,583,924; U.S. Patent No. 8,584,945;
U.S. Patent No. 8,587,595; U.S. Patent No. 8,587,697;
U.S. Patent No. 8,588,869; U.S. Patent No. 8,590,789;
U.S. Patent No. 8,596,539; U.S. Patent No. 8,596,542;
U.S. Patent No. 8,596,543; U.S. Patent No. 8,599,271;
U.S. Patent No. 8,599,957; U.S. Patent No. 8,600,158;
U.S. Patent No. 8,600,167; U.S. Patent No. 8,602,309;
U.S. Patent No. 8,608,053; U.S. Patent No. 8,608,071;
U.S. Patent No. 8,611,309; U.S. Patent No. 8,615,487;
U.S. Patent No. 8,616,454; U.S. Patent No. 8,621,123;
U.S. Patent No. 8,622,303; U.S. Patent No. 8,628,013;
U.S. Patent No. 8,628,015; U.S. Patent No. 8,628,016;
U.S. Patent No. 8,629,926; U.S. Patent No. 8,630,491;
U.S. Patent No. 8,635,309; U.S. Patent No. 8,636,200;
U.S. Patent No. 8,636,212; U.S. Patent No. 8,636,215;
U.S. Patent No. 8,636,224; U.S. Patent No. 8,638,806;
U.S. Patent No. 8,640,958; U.S. Patent No. 8,640,960;
U.S. Patent No. 8,643,717; U.S. Patent No. 8,646,692;
U.S. Patent No. 8,646,694; U.S. Patent No. 8,657,200;
U.S. Patent No. 8,659,397; U.S. Patent No. 8,668,149;
U.S. Patent No. 8,678,285; U.S. Patent No. 8,678,286;
U.S. Patent No. 8,682,077; U.S. Patent No. 8,687,282;
U.S. Patent No. 8,692,927; U.S. Patent No. 8,695,880;
U.S. Patent No. 8,698,949; U.S. Patent No. 8,717,494;
U.S. Patent No. 8,717,494; U.S. Patent No. 8,720,783;
U.S. Patent No. 8,723,804; U.S. Patent No. 8,723,904;
U.S. Patent No. 8,727,223; U.S. Patent No. D702,237;
U.S. Patent No. 8,740,082; U.S. Patent No. 8,740,085;
U.S. Patent No. 8,746,563; U.S. Patent No. 8,750,445;
U.S. Patent No. 8,752,766; U.S. Patent No. 8,756,059;
U.S. Patent No. 8,757,495; U.S. Patent No. 8,760,563;
U.S. Patent No. 8,763,909; U.S. Patent No. 8,777,108;
U.S. Patent No. 8,777,109; U.S. Patent No. 8,779,898;
U.S. Patent No. 8,781,520; U.S. Patent No. 8,783,573;
U.S. Patent No. 8,789,757; U.S. Patent No. 8,789,758;
U.S. Patent No. 8,789,759; U.S. Patent No. 8,794,520;
U.S. Patent No. 8,794,522; U.S. Patent No. 8,794,526;
U.S. Patent No. 8,798,367; U.S. Patent No. 8,807,431;
U.S. Patent No. 8,807,432; U.S. Patent No. 8,820,630;
International Publication No. 2013/163789;
International Publication No. 2013/173985;
International Publication No. 2014/019130;
International Publication No. 2014/110495;
U.S. Patent Application Publication No. 2008/0185432;
U.S. Patent Application Publication No. 2009/0134221;
U.S. Patent Application Publication No. 2010/0177080;
U.S. Patent Application Publication No. 2010/0177076;
U.S. Patent Application Publication No. 2010/0177707;
U.S. Patent Application Publication No. 2010/0177749;
U.S. Patent Application Publication No. 2011/0202554;
U.S. Patent Application Publication No. 2012/0111946;
U.S. Patent Application Publication No. 2012/0138685;
U.S. Patent Application Publication No. 2012/0168511;
U.S. Patent Application Publication No. 2012/0168512;
U.S. Patent Application Publication No. 2012/0193423;
U.S. Patent Application Publication No. 2012/0203647;
U.S. Patent Application Publication No. 2012/0223141;
U.S. Patent Application Publication No. 2012/0228382;
U.S. Patent Application Publication No. 2012/0248188;
U.S. Patent Application Publication No. 2013/0043312;
U.S. Patent Application Publication No. 2013/0056285;
U.S. Patent Application Publication No. 2013/0070322;
U.S. Patent Application Publication No. 2013/0075168;
U.S. Patent Application Publication No. 2013/0082104;
U.S. Patent Application Publication No. 2013/0175341;
U.S. Patent Application Publication No. 2013/0175343;
U.S. Patent Application Publication No. 2013/0200158;
U.S. Patent Application Publication No. 2013/0256418;
U.S. Patent Application Publication No. 2013/0257744;
U.S. Patent Application Publication No. 2013/0257759;
U.S. Patent Application Publication No. 2013/0270346;
U.S. Patent Application Publication No. 2013/0278425;
U.S. Patent Application Publication No. 2013/0287258;
U.S. Patent Application Publication No. 2013/0292475;
U.S. Patent Application Publication No. 2013/0292477;
U.S. Patent Application Publication No. 2013/0293539;
U.S. Patent Application Publication No. 2013/0293540;
U.S. Patent Application Publication No. 2013/0306728;
U.S. Patent Application Publication No. 2013/0306730;
U.S. Patent Application Publication No. 2013/0306731;
U.S. Patent Application Publication No. 2013/0307964;
U.S. Patent Application Publication No. 2013/0308625;
U.S. Patent Application Publication No. 2013/0313324;
U.S. Patent Application Publication No. 2013/0313325;
U.S. Patent Application Publication No. 2013/0341399;
U.S. Patent Application Publication No. 2013/0342717;
U.S. Patent Application Publication No. 2014/0001267;
U.S. Patent Application Publication No. 2014/0002828;
U.S. Patent Application Publication No. 2014/0008430;
U.S. Patent Application Publication No. 2014/0008439;
U.S. Patent Application Publication No. 2014/0025584;
U.S. Patent Application Publication No. 2014/0027518;
U.S. Patent Application Publication No. 2014/0034734;
U.S. Patent Application Publication No. 2014/0036848;
U.S. Patent Application Publication No. 2014/0039693;
U.S. Patent Application Publication No. 2014/0042814;
U.S. Patent Application Publication No. 2014/0049120;
U.S. Patent Application Publication No. 2014/0049635;
U.S. Patent Application Publication No. 2014/0061305;
U.S. Patent Application Publication No. 2014/0061306;
U.S. Patent Application Publication No. 2014/0063289;
U.S. Patent Application Publication No. 2014/0066136;
U.S. Patent Application Publication No. 2014/0067692;
U.S. Patent Application Publication No. 2014/0070005;
U.S. Patent Application Publication No. 2014/0071840;
U.S. Patent Application Publication No. 2014/0074746;
U.S. Patent Application Publication No. 2014/0075846;
U.S. Patent Application Publication No. 2014/0076974;
U.S. Patent Application Publication No. 2014/0078341;
U.S. Patent Application Publication No. 2014/0078342;
U.S. Patent Application Publication No. 2014/0078345;
U.S. Patent Application Publication No. 2014/0084068;
U.S. Patent Application Publication No. 2014/0097249;
U.S. Patent Application Publication No. 2014/0098792;
U.S. Patent Application Publication No. 2014/0100774;
U.S. Patent Application Publication No. 2014/0100813;
U.S. Patent Application Publication No. 2014/0103115;
U.S. Patent Application Publication No. 2014/0104413;
U.S. Patent Application Publication No. 2014/0104414;
U.S. Patent Application Publication No. 2014/0104416;
U.S. Patent Application Publication No. 2014/0104451;
U.S. Patent Application Publication No. 2014/0106594;
U.S. Patent Application Publication No. 2014/0106725;
U.S. Patent Application Publication No. 2014/0108010;
U.S. Patent Application Publication No. 2014/0108402;
U.S. Patent Application Publication No. 2014/0108682;
U.S. Patent Application Publication No. 2014/0110485;
U.S. Patent Application Publication No. 2014/0114530;
U.S. Patent Application Publication No. 2014/0124577;
U.S. Patent Application Publication No. 2014/0124579;
U.S. Patent Application Publication No. 2014/0125842;
U.S. Patent Application Publication No. 2014/0125853;
U.S. Patent Application Publication No. 2014/0125999;
U.S. Patent Application Publication No. 2014/0129378;
U.S. Patent Application Publication No. 2014/0131438;
U.S. Patent Application Publication No. 2014/0131441;
U.S. Patent Application Publication No. 2014/0131443;
U.S. Patent Application Publication No. 2014/0131444;
U.S. Patent Application Publication No. 2014/0131445;
U.S. Patent Application Publication No. 2014/0131448;
U.S. Patent Application Publication No. 2014/0133379;
U.S. Patent Application Publication No. 2014/0136208;
U.S. Patent Application Publication No. 2014/0140585;
U.S. Patent Application Publication No. 2014/0151453;
U.S. Patent Application Publication No. 2014/0152882;
U.S. Patent Application Publication No. 2014/0158770;
U.S. Patent Application Publication No. 2014/0159869;
U.S. Patent Application Publication No. 2014/0160329;
U.S. Patent Application Publication No. 2014/0166755;
U.S. Patent Application Publication No. 2014/0166757;
U.S. Patent Application Publication No. 2014/0166759;
U.S. Patent Application Publication No. 2014/0166760;
U.S. Patent Application Publication No. 2014/0166761;
U.S. Patent Application Publication No. 2014/0168787;
U.S. Patent Application Publication No. 2014/0175165;
U.S. Patent Application Publication No. 2014/0175169;
U.S. Patent Application Publication No. 2014/0175172;
U.S. Patent Application Publication No. 2014/0175174;
U.S. Patent Application Publication No. 2014/0191644;
U.S. Patent Application Publication No. 2014/0191913;
U.S. Patent Application Publication No. 2014/0197238;
U.S. Patent Application Publication No. 2014/0197239;
U.S. Patent Application Publication No. 2014/0197304;
U.S. Patent Application Publication No. 2014/0203087;
U.S. Patent Application Publication No. 2014/0204268;
U.S. Patent Application Publication No. 2014/0214631;
U.S. Patent Application Publication No. 2014/0217166;
U.S. Patent Application Publication No. 2014/0217180;
U.S. Patent Application No. 13/367,978 for a Laser Scanning Module Employing an Elastomeric U-Hinge Based Laser Scanning Assembly, filed February 7, 2012 (Feng et al.*);*
U.S. Patent Application No. 29/436,337 for an Electronic Device, filed November 5, 2012 (Fitch et al.*);*
U.S. Patent Application No. 13/771,508 for an Optical Redirection Adapter, filed February 20, 2013 (Anderson);
U.S. Patent Application No. 13/852,097 for a System and Method for Capturing and Preserving Vehicle Event Data, filed March 28, 2013 (Barker et al.);
U.S. Patent Application No. 13/902,110 for a System and Method for Display of Information Using a Vehicle-Mount Computer, filed May 24, 2013 (Hollifield);
U.S. Patent Application No. 13/902,144, for a System and Method for Display of Information Using a Vehicle-Mount Computer, filed May 24, 2013 (Chamberlin);
U.S. Patent Application No. 13/902,242 for a System For Providing A Continuous Communication Link Witch A Symbol Reading Device, filed May 24, 2013 (Smith et al.);
U.S. Patent Application No. 13/912,262 for a Method of Error Correction for 3D Imaging Device, filed June 7, 2013 (Jovanovski et al.*);*
U.S. Patent Application No. 13/912,702 for a System and Method for Reading Code Symbols at Long Range Using Source Power Control, filed June 7, 2013 (Xian et al.);
U.S. Patent Application No. 29/458,405 for an Electronic Device, filed June 19, 2013 (Fitch et al.*);*
U.S. Patent Application No. 13/922,339 for a System and Method for Reading Code Symbols Using a Variable Field of View, filed June 20, 2013 (Xian et al.);
U.S. Patent Application No. 13/927,398 for a Code Symbol Reading System Having Adaptive Autofocus, filed June 26, 2013 (Todeschini);
U.S. Patent Application No. 13/930,913 for a Mobile Device Having an Improved User Interface for Reading Code Symbols, filed June 28, 2013 (Gelay et al.);
U.S. Patent Application No. 29/459,620 for an Electronic Device Enclosure, filed July 2, 2013 (London et al.*);*
U.S. Patent Application No. 29/459,681 for an Electronic Device Enclosure, filed July 2, 2013 (Chaney et al.*);*
U.S. Patent Application No. 13/933,415 for an Electronic Device Case, filed July 2, 2013 (London et al.*);*
U.S. Patent Application No. 29/459,785 for a Scanner and Charging Base, filed July 3, 2013 (Fitch et al.);
U.S. Patent Application No. 29/459,823 for a Scanner, filed July 3, 2013 (Zhou et al.);
U.S. Patent Application No. 13/947,296 for a System and Method for Selectively Reading Code Symbols, filed July 22, 2013 (Rueblinger et al.*);*
U.S. Patent Application No. 13/950,544 for a Code Symbol Reading System Having Adjustable Object Detection, filed July 25, 2013 (Jiang);
U.S. Patent Application No. 13/961,408 for a Method for Manufacturing Laser Scanners, filed August 7, 2013 (Saber et al.*);*
U.S. Patent Application No. 14/018,729 for a Method for Operating a Laser Scanner, filed September 5, 2013 (Feng et al.*);*
U.S. Patent Application No. 14/019,616 for a Device Having Light Source to Reduce Surface Pathogens, filed September 6, 2013 (Todeschini);
U.S. Patent Application No. 14/023,762 for a Handheld Indicia Reader Having Locking Endcap, filed September 11, 2013 (Gannon);
U.S. Patent Application No. 14/035,474 for Augmented-Reality Signature Capture, filed September 24, 2013 (Todeschini);
U.S. Patent Application No. 29/468,118 for an Electronic Device Case, filed September 26, 2013 (Oberpriller et al.*);*
U.S. Patent Application No. 14/055,234 for Dimensioning System, filed October 16, 2013 (Fletcher);
U.S. Patent Application No. 14/053,314 for Indicia Reader, filed October 14, 2013 (Huck);
U.S. Patent Application No. 14/065,768 for Hybrid System and Method for Reading Indicia, filed October 29, 2013 (Meier et al.*);*
U.S. Patent Application No. 14/074,746 for Self-Checkout Shopping System, filed November 8, 2013 (Hejl et al.*);*
U.S. Patent Application No. 14/074,787 for Method and System for Configuring Mobile Devices via NFC Technology, filed November 8, 2013 (Smith et al.);
U.S. Patent Application No. 14/087,190 for Optimal Range Indicators for Bar Code Validation, filed November 22, 2013 (Hejl);
U.S. Patent Application No. 14/094,087 for Method and System for Communicating Information in an Digital Signal, filed December 2, 2013 (Peake et al.)*;*
U.S. Patent Application No. 14/101,965 for High Dynamic-Range Indicia Reading System, filed December 10, 2013 (Xian);
U.S. Patent Application No. 14/150,393 for Indicia-reader Having Unitary Construction Scanner, filed January 8, 2014 (Colavito et al.);
U.S. Patent Application No. 14/154,207 for Laser Barcode Scanner, filed January 14, 2014 (Hou et al.);
U.S. Patent Application No. 14/165,980 for System and Method for Measuring Irregular Objects with a Single Camera filed January 28, 2014 (Li et al.);
U.S. Patent Application No. 14/166,103 for Indicia Reading Terminal Including Optical Filter filed January 28, 2014 (Lu et al.);
U.S. Patent Application No. 14/200,405 for Indicia Reader for Size-Limited Applications filed March 7, 2014 (Feng et al.);
U.S. Patent Application No. 14/231,898 for Hand-Mounted Indicia-Reading Device with Finger Motion Triggering filed April 1, 2014 (Van Horn et al.);
U.S. Patent Application No. 14/250,923for Reading Apparatus Having Partial Frame Operating Mode filed April 11, 2014, (Deng et al.*);*
U.S. Patent Application No. 14/257,174 for Imaging Terminal Having Data Compression filed April 21, 2014, (Barber et al.*);*
U.S. Patent Application No. 14/257,364 for Docking System and Method Using Near Field Communication filed April 21, 2014 (Showering);
U.S. Patent Application No. 14/264,173 for Autofocus Lens System for Indicia Readers filed April 29, 2014 (Ackley et al.*);*
U.S. Patent Application No. 14/274,858 for Mobile Printer with Optional Battery Accessory filed May 12, 2014 (Marty et al.*);*
U.S. Patent Application No. 14/277,337 for MULTIPURPOSE OPTICAL READER, filed May 14, 2014 (Jovanovski et al.*);*
U.S. Patent Application No. 14/283,282 for TERMINAL HAVING ILLUMINATION AND FOCUS CONTROL filed May 21, 2014 (Liu et al.*);*
U.S. Patent Application No. 14/300,276 for METHOD AND SYSTEM FOR CONSIDERING INFORMATION ABOUT AN EXPECTED RESPONSE WHEN PERFORMING SPEECH RECOGNITION, filed June 10, 2014 (Braho et al.*);*
U.S. Patent Application No. 14/305,153 for INDICIA READING SYSTEM EMPLOYING DIGITAL GAIN CONTROL filed June 16, 2014 (Xian et al.*);*
U.S. Patent Application No. 14/310,226 for AUTOFOCUSING OPTICAL IMAGING DEVICE filed June 20, 2014 (Koziol et al.*);*
U.S. Patent Application No. 14/327,722 for CUSTOMER FACING IMAGING SYSTEMS AND METHODS FOR OBTAINING IMAGES filed July 10, 2014 (Oberpriller et al*,);*
U.S. Patent Application No. 14/327,827 for a MOBILE-PHONE ADAPTER FOR ELECTRONIC TRANSACTIONS, filed July 10, 2014 (Hejl);
U.S. Patent Application No. 14/329,303 for CELL PHONE READING MODE USING IMAGE TIMER filed July 11, 2014 (Coyle);
U.S. Patent Application No. 14/333,588 for SYMBOL READING SYSTEM WITH INTEGRATED SCALE BASE filed July 17, 2014 (Barten);
U.S. Patent Application No. 14/334,934 for a SYSTEM AND METHOD FOR INDICIA VERIFICATION, filed July 18, 2014 (Hejl);
U.S. Patent Application No. 14/336,188 for METHOD OF AND SYSTEM FOR DETECTING OBJECT WEIGHING INTERFERENCES, Filed July 21, 2014 (Amundsen et al.);
U.S. Patent Application No. 14/339,708 for LASER SCANNING CODE SYMBOL READING SYSTEM, filed July 24, 2014 (Xian et al.);
U.S. Patent Application No. 14/340,627 for an AXIALLY REINFORCED FLEXIBLE SCAN ELEMENT, filed July 25, 2014 (Rueblinger et al.);
U.S. Patent Application No. 14/340,716 for an OPTICAL IMAGER AND METHOD FOR CORRELATING A MEDICATION PACKAGE WITH A PATIENT, filed July 25, 2014 (Ellis);
U.S. Patent Application No. 14/342,544 for Imaging Based Barcode Scanner Engine with Multiple Elements Supported on a Common Printed Circuit Board filed March 4, 2014 (Liu et al.*);*
U.S. Patent Application No. 14/345,735 for Optical Indicia Reading Terminal with Combined Illumination filed March 19, 2014 (Ouyang);
U.S. Patent Application No. 14/336,188 for METHOD OF AND SYSTEM FOR DETECTING OBJECT WEIGHING INTERFERENCES, Filed July 21, 2014 (Amundsen et al.);
U.S. Patent Application No. 14/355,613 for Optical Indicia Reading Terminal with Color Image Sensor filed May 1, 2014 (Lu et al.*);*
U.S. Patent Application No. 14/370,237 for WEB-BASED SCAN-TASK ENABLED SYSTEM AND METHOD OF AND APPARATUS FOR DEVELOPING AND DEPLOYING THE SAME ON A CLIENT-SERVER NETWORK filed 07/02/2014 (Chen et al.*);*
U.S. Patent Application No. 14/370,267 for INDUSTRIAL DESIGN FOR CONSUMER DEVICE BASED SCANNING AND MOBILITY, filed July 2, 2014 (Ma et al.*);*
U.S. Patent Application No. 14/376,472, for an ENCODED INFORMATION READING TERMINAL INCLUDING HTTP SERVER, filed 08-04-2014 (Lu*) ;*
U.S. Patent Application No. 14/379,057 for METHOD OF USING CAMERA SENSOR INTERFACE TO TRANSFER MULTIPLE CHANNELS OF SCAN DATA USING AN IMAGE FORMAT filed August 15, 2014 (Wang et al.*);*
U.S. Patent Application No. 14/452,697 for INTERACTIVE INDICIA READER, filed August 6, 2014 (Todeschini);
U.S. Patent Application No. 14/453,019 for DIMENSIONING SYSTEM WITH GUIDED ALIGNMENT, filed August 6, 2014 (Li et al.);
U.S. Patent Application No. 14/460,387 for APPARATUS FOR DISPLAYING BAR CODES FROM LIGHT EMITTING DISPLAY SURFACES filed August 15, 2014 (Van Horn et al.);
U.S. Patent Application No. 14/460,829 for ENCODED INFORMATION READING TERMINAL WITH WIRELESS PATH SELECTON CAPABILITY, filed August 15, 2014 (Wang et al.);
U.S. Patent Application No. 14/462,801 for MOBILE COMPUTING DEVICE WITH DATA COGNITION SOFTWARE, filed on August 19, 2014 (Todeschini et al.);
U.S. Patent Application No.14/446,387 for INDICIA READING TERMINAL PROCESSING PLURALITY OF FRAMES OF IMAGE DATA RESPONSIVELY TO TRIGGER SIGNAL ACTIVATION filed July 30, 2014 (Wang et al.);
U.S. Patent Application No. 14/446,391 for MULTIFUNCTION POINT OF SALE APPARATUS WITH OPTICAL SIGNATURE CAPTURE filed July 30, 2014 (Good et al.);
U.S. Patent Application No. 29/486,759 for an Imaging Terminal, filed April 2, 2014 (Oberpriller et al.);
U.S. Patent Application No. 29/492,903 for an INDICIA SCANNER, filed June 4, 2014 (Zhou et al.); and
U.S. Patent Application No. 29/494,725 for an IN-COUNTER BARCODE SCANNER, filed June 24, 2014 (Oberpriller et al.).

In the specification and/or figures, typical embodiments of the invention have been disclosed. The present invention is not limited to such exemplary embodiments. The use of the term "and/or" includes any and all combinations of one or more of the associated listed items. The figures are schematic representations and so are not necessarily drawn to scale. Unless otherwise noted, specific terms have been used in a generic and descriptive sense and not for purposes of limitation.

## Claims

1. A system for medication management, comprising:
a camera;
a user interface, comprising a visual display; and
one or more processors communicatively coupled to the camera and the user interface system, the one or more processors being configured to:
capture from the camera an image depicting a machine readable code symbol, where the code symbol associates a medication with a patient;
determine prescribing information for the medication and the patient;
determine that a dose of medication is due; and
generate an alert indicating that the dose of medication is due to be taken by the patient.

2. The system according to claim 1, further comprising a medication dispenser; and
where the one or more processors are further configured to cause the medication dispenser to provide patient access to a dose of medication when the medication is due to be taken by the patient.

3. The system according to claim 2, where the one or more processors are further configured to create entries to a log that record times and instances of alerts and dispenses of medication.

4. The system according to claim 1, further comprising a medication dispenser; and
where the one or more processors are further configured to:
authenticate the presence of the patient; and
cause the medication dispenser to provide patient access to a dose of medication when the medication is due to be taken by the patient and when the patient is authenticated.

5. The system according to claim 3, where the one or more processors are further configured to create a log entry that records times and instances of alerts, authentications, and medication dispenses.

6. The system according to claim 1, where the one or more processors are further configured to retrieve information relating to the medication.

7. The system according to claim 6, where the information is retrieved from a database residing in the one or more processors' directly accessible memory.

8. The system according to claim 6, where the information is retrieved from a database residing outside the one or more processors' directly accessible memory.

9. The system according to claim 8, where the database is accessed via a wireless communication system.

10. The system according to claim 1, where the one or more processors are further configured to:
receive acknowledgements of alerts by the patient;
create a log entry that records time and instances of alerts and acknowledgements of alerts.

11. The system according to claim 1, where the code symbol comprises a bar code.

12. A method of medication management, comprising:
at a camera, capturing an image depicting a machine readable code symbol, where the code symbol associates a medication with a patient;
at one or more programmed processors:
determining prescribing information for the medication and the patient;
determining that a dose of the medication is due; and
generating an alert indicating that the dose of medication is due to be taken by the patient.

13. The method according to claim 12, further comprising the one or more processors causing a medication dispenser to provide patient access to a dose of medication when the medication is due to be taken by the patient.

14. The method according to claim 12, further comprising the one or more processors creating entries to a log that records times and instances of alerts and dispenses of medication.

15. The method according to claim 12, further comprising:
the one or more processors authenticating the presence of the patient;
the one or more processors causing a medication dispenser to provide patient access to a dose of medication when the medication is due to be taken by the patient and when the patient is authenticated.
